# EUROPÄISCHE PATENTSCHRIFT

(11) **EP 3 121 595 B1**
(45) Veröffentlichungstag und Bekanntmachung des Hinweises auf die Patenterteilung: **06.12.2017**
(21) Anmeldenummer: 15177929.5
(22) Anmeldetag: 22.07.2015
(51) Int. Cl.: G01N 33/28, G01N 21/64, G01N 15/06, G01N 21/77

(54) **VERFAHREN ZUR ERMITTLUNG DES GEHALTS AN CATALYST FINES IN EINER PROBE EINES SCHWERÖLS**
METHOD FOR DETERMINING THE CONTENT OF CATALYST FINES IN A SAMPLE OF A HEAVY FUEL OIL
PROCEDE DE DETERMINATION DE TENEUR EN FINES PARTICULES CATALYTIQUES DANS UN ECHANTILLON DE FIOUL LOURD

(43) Veröffentlichungstag der Anmeldung: 25.01.2017
(73) Patentinhaber: CM Technologies GmbH, 35337 Elmshorn (DE)
(72) Erfinder: Jeske, Andreas, 25335 Elmshorn (DE); Winkler, Matthias, 25337 Elmshorn (DE)
(74) Vertreter: Meyer, Ludgerus

(56) Entgegenhaltungen:
- JP-A- 2005 030 815
- US-B1- 7 286 633

## Beschreibung

Die Erfindung bezieht sich auf ein Verfahren zur Ermittlung des Gehalts an Catalyst Fines in einer Probe eines Schweröls, insbesondere eines Kraftstoffes für Schiffsmotoren.

Auf Seeschiffen, insbesondere für den internationalen Gütertransport, sind die Antriebsaggregate in der Regel als Dieselmotoren ausgeführt, denen als Brennstoff Schweröle zugeführt werden. Diese sind hauptsächlich aus Rückständen der Erdölverarbeitung hergestellt. Während der Destillation von Rohöl in Erdölraffinerien werden beim sogenannten katalytischen Cracken Katalysatoren verwendet, die Catalyst Fines (Catfines) enthalten. Dabei handelt es sich hauptsächlich um Aluminium-Siliziumoxide (Al₂O₃ und SiO₂). Diese sind sehr hart und abrasiv. Sie verbleiben nach dem Cracken zum Teil in den Rückstandsölen, die dann als Kraftstoffe für die Schiffsdieselmotoren verwendet werden und stellen damit eine erhebliche Gefahr für verschiedene Bauteile des Einspritzsystems, den Kolbenringen und den Zylinderbuchsen dar. Vor der Verwendung derartiger Kraftstoffe ist daher eine gründliche Aufbereitung erforderlich, die auch eine Filtration des Kraftstoffs beinhaltet.

In der ISO Norm 8217 ist für die Lieferung von Schweröl ein maximaler Gehalt von 60 ppm Catfines vorgeschrieben. Dagegen schreiben Motorenhersteller einen maximalen Gehalt von 10 - 15 ppm Catfines vor Eintritt in den Motor vor.

Neben der Filtration des Kraftstoffs kann die Abtrennung von Catfines aus den Schwerölen auch mittels Zentrifugen vorgenommen werden. Ein entsprechendes Verfahren und ein System zur Abtrennung von Catalyst Fines aus einem Ölstrom ist in der WO 2014/001168 A1 angegeben. In Abhängigkeit von dem durch ein NMR-Verfahren erfassten Catfines-Gehalt im Schweröl erfolgt mittels der Zentrifuge eine mehr oder weniger starke Reinigung des Schweröls.

Ein solches Verfahren kann zwar den Gehalt am Catalyst Fines im Schweröl reduzieren, jedoch ist die Feststellung des Gehalts am Catalyst Fines im Schweröl mittels einer NMR-Spektroskopie auf einem Seeschiff normalerweise nicht möglich und zu aufwendig.

Ferner ist aus JP2005030815A bekannt, Catalyst Fines aus dem Öl zu filtern und mittels eines Mikroskopes zu untersuchen.

Weiterhin ist aus US7286633B1 bekannt, Catalyst Fines mittels Röntgenfluoreszenzspektroskopie (XRF) zu bestimmen.

Der Erfindung liegt daher die Aufgabe zugrunde, ein einfaches Verfahren zur Ermittlung des Gehalts an Catalyst Fines in einem Schweröl, insbesondere eines Kraftstoffs für Schiffsmotoren, anzugeben, das vor Ort auf einem Schiff verwendbar ist und eine praxisgerechte Aussage über den Gehalt an Catalyst Fines in einem Schweröl liefert.

Diese Aufgabe wird durch die im Anspruch 1 angegebene Erfindung gelöst. Vorteilhafte Weiterbildungen der Erfindung sind in Unteransprüchen angegeben.

Gemäß dem erfindungsgemäßen Verfahren wird eine verdünnte Probe eines Schweröls mit Salzsäure zur oberflächlichen Ionisierung von Aluminium aus den Catfines vermischt. Dieses ionisierte dreiwertige Aluminium-Ion verhält sich in wässrigen Lösungen amphoter: es bildet mit wässrigen Bestandteilen pH-Wert abhängig unterschiedliche Komplexe. Durch die Anregung dieser Komplexe mittels UV-Licht und einer speziellen Detektionswellenlänge kann die Schwächung der Strahlung im Spektrum erkannt werden.

Da das Verhältnis zwischen Aluminiumoxide und Siliziumoxide in den Catfines ungefähr gleich ist, kann allein aus der Feststellung des Aluminiumgehalts der gesamte Catfines-Gehalt in einer Probe des Schweröls ermittelt werden.

Insbesondere wird bei dem erfindungsgemäßen Verfahren zunächst eine Probe eines Schweröls mit einem ersten Reagenz, das hauptsächlich aus Kohlenwasserstoffen besteht und üblicherweise als WBC-Verdünner bezeichnet wird, verdünnt und intensiv vermischt. Dadurch wird die Zugänglichkeit zu den Aluminiumsilikaten der Catfines gesichert. Ein Teil der Mischung aus dem ersten Behälter wird in einen zweiten verdünnte Salzsäure als zweites Reagenz enthaltenden zweiten Behälter eingebracht und mit der Salzsäure intensiv vermischt, um eine oberflächliche Ionisierung des Aluminiums aus den Catfines zu erreichen und eine Pufferung im Bereich von pH 2 - 4 zu erzielen.

Ein Teil des Inhalts des zweiten Behälters wird dann über einen Filter in einen dritten Behälter überführt, in dem die Feststellung des Gehalts an Catalyst Fines in der Probe bei UV-Lichtanregung durch Ermittlung der Stärke der sich ergebenden Fluoreszenzstrahlung visuell oder mit Hilfe eines Photometers erfasst wird.

Die Quantifizierung erfolgt somit im sauren Bereich durch Bildung von Komplexe wie folgt:

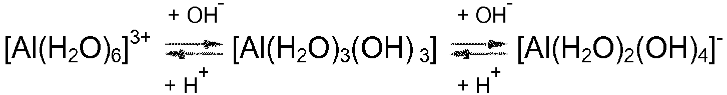

Die Formel beschreibt ein pH-Wert abhängiges amphoteres Verhalten von Aluminium-Ionen in Wasser mit der Folge der Komplexbildung.
Links: dreiwertiger Komplex im stark sauren Bereich
Mitte: neutraler Komplex bei pH = 7
Rechts: einfach negativ geladener Komplex im stark basischen Bereich

Eine Quantifizierung ist somit auch im basischen Bereich möglich, wenn statt der Salzsäure als zweites Reagenz Kaliumhydroxid (KOH) verwendet wird.

Als Verdünner für das Schweröl wird vorzugsweise ein WBC6-Verdünner verwendet, bei dem es sich um einen Standardverdünner in Form eines Universalkaltreinigers handelt, der aus aliphatischen Kohlenwasserstoffen mit n-Alkanen, C10-C13- Verbindungen, iso-Alkanen und Cycloalkanen besteht.

Das zweite Reagenz ist vorzugsweise in doppelt destilliertem Wasser verdünnte 0,05 molare Salzsäurelösung.

Die Entnahme aus dem zweiten Behälter erfolgt vorzugsweise mit einer 5 ml Kolbenspritze, der ein mit verdünnter Salzsäure vorgespülter Filter vorgesetzt ist.

Die Durchmischung der Probe und der Reagenzien erfolgt vorzugsweise mittels eines Vortexmischers.

Zur Beschleunigung des Verfahrens kann die Vermischung auch mit erhöhter Temperatur unter Verwendung von Ultraschall durchgeführt werden.

Die Feststellung des Gehalts in Catalyst Fines in der Probe erfolgt vorzugsweise durch Anregung mittels einer UV-Diode bei einer Wellenlänge von 365 nm. Die sich ergebende Fluoreszenzstrahlung kann bei 470 nm kann visuell erfasst werden oder vorzugsweise mittels eines Photometers ausgewertet werden.

Das erfindungsgemäße Verfahren kann vor Ort auf einem Schiff mit einfachen Mitteln durchgeführt werden, so dass eine ständige Kontrolle der Qualität des Schweröls möglich ist, sofern nicht bereits bei Bunkerung des Schweröls eine entsprechende Prüfung durchgeführt wurde. Da Catfines jedoch die Tendenz haben, in einem Schwerölbunker einen Bodensatz zu bilden, ist es von Vorteil, die Analyse des Schweröls während einer Fahrt mehrfach zu wiederholen.

In einem beispielhaft durchgeführten Verfahren wird Schweröl im Verhältnis von 2:1, vorzugsweise 8 ml Schweröl und 4 ml WBC-Verdünner, in einem ersten Behälter mittels eines Vortexmischers für 1 - 3 Minuten gemischt. Mit einer Kolbenspritze werden 7 ml Salzsäurelösung, gepuffert im pH-Bereich von 2 - 4, aufgenommen, wovon 2 ml zur Befeuchtung eines Filters angewandt und die verbbleibenden 5 ml in einen zweiten Behälter filtriert werden. Dann werden 0,2 ml der verdünnten Schwerölprobe aus Behältnis 1 in den zweiten Behälter übergeben. Der zweite Behälter wird nun für 30 Sekunden mittels des Vortexmischers vermischt. Aus dem zweiten Behälter werden dann mittels einer Kolbenspritze 4 ml der Mischung aufgenommen. Diese 4 ml werden durch den zuvor benetzten Filter in ein drittes Behältnis gegeben. Der dritte Behälter wird nun in ein Photometer gestellt, wobei er mit einer UV-Diode mit 365 nm Wellenlänge be- und durchstrahlt wird. Die resultierende Änderung der Strahlung kann bei 470 nm ausgewertet und über eine elektronische Schaltung an ein Anzeigegerät übergeben werden, oder es wird ein Protokoll mit den gemessenen Werten erstellt. Der gefundene Wert entspricht dem Gehalt an Aluminium in der Schwerölprobe und repräsentiert auch den Gesamtgehalt an Catfines.

## Patentansprüche

1. Verfahren zur Ermittlung des Gehalts an Catalyst Fines in einer Probe eines Schweröls, insbesondere eines Kraftstoffes für Schiffsmotoren, mittels folgender Schritte:
a) aus dem Schweröl wird eine Probe entnommen,
b) die Probe wird in einem ersten Behälter mit einem ersten Reagenz aus hauptsächlich Kohlenwasserstoffen enthaltenden Lösungsmittel verdünnt und intensiv vermischt,
c) ein Teil der Mischung des ersten Behälters wird in einen zweiten verdünnte Salzsäure als zweites Reagenz enthaltenden zweiten Behälter eingebracht und mit der Salzsäure intensiv vermischt,
d) aus dem zweiten Behälter wird ein Teil des Inhalts entnommen und über einen Filter in einen dritten Behälter überführt,
e) im dritten Behälter erfolgt die Feststellung des Gehalts an Catalyst Fines in der Probe durch Ermittlung der Stärke der sich ergebenden Fluoreszenzstrahlung bei UV-Lichtanregung
f) die sich zeigende Fluoreszenzstrahlung wird visuell oder mit Hilfe eines Photometers erfasst und bewertet.

2. Verfahren nach Anspruch 1, **dadurch gekennzeichnet, dass** das erste Reagenz ein Standardverdünner in Form eines Universalkaltreinigers ist, der aus aliphatischen Kohlenwasserstoffen mit n-Alkanen, C₁₀-C₁₃ - Verbindungen, iso-Alkanen und Cycloalkanen besteht.

3. Verfahren nach Anspruch 1, **dadurch gekennzeichnet, dass** das zweite Reagenz mit doppelt destilliertem Wasser verdünnte 0,05 molare Salzsäurelösung ist.

4. Verfahren nach Anspruch 1, **dadurch gekennzeichnet, dass** die Entnahme aus dem zweiten Behälter mittels einer Kolbenspritze erfolgt, deren Inhalt durch einen mit verdünnter Salzsäure benetzten Filter abgegeben wird.

5. Verfahren nach Anspruch 1, **dadurch gekennzeichnet, dass** die Vermischung gemäß Schritt c) mittels eines Vortexmischers bei Umgebungstemperatur durchgeführt wird.

6. Verfahren nach Anspruch 1, **dadurch gekennzeichnet, dass** die Vermischung gemäß Schritt c) mittels Ultraschall bei erhöhter Temperatur durchgeführt wird.

7. Verfahren nach Anspruch 1, **dadurch gekennzeichnet, dass** die Anregung durch UV-Strahlung mittels wenigstens einer UV-Diode bei 365 nm erfolgt.

8. Verfahren nach Anspruch 7, **dadurch gekennzeichnet, dass** die Stärke der Fluoreszenzstrahlung bei 470 nm ermittelt wird.

9. Verfahren nach Anspruch 4, **dadurch gekennzeichnet, dass** der Filter vor seiner Verwendung mit einer 0,05 molaren Salzsäurelösung bei pH-Stabilisierung im Bereich von 2 - 4 vorgespült wird.

10. Verfahren nach Anspruch 2, **dadurch gekennzeichnet, dass** in Schritt b) Schweröl (HFO) mit einem Verdünner nach Anspruch 2 im Volumenverhältnis 2:1 für 2 - 4 Minuten im ersten Behälter mittels eines Vortexmischers gemischt werden, dass in Schritt c) 5 ml des zweiten Reagenz im zweiten Behälter mit 0,2 ml der Mischung aus dem ersten Behälter mittels eines Vortexmischers für 20 - 40 Sekunden vermischt werden, dass in Schritt d) 3 - 5 ml entnommen und gefiltert werden und das Filtrat in den dritten Behälter überführt wird, in dem die Ermittlung der Fluoreszenzstrahlung gemäß Schritt f) erfolgt.

11. Verfahren nach Anspruch 1, **dadurch gekennzeichnet, dass** das in Schritt c) aus dem zweiten Behälter entnommene Filtrat auf einen pH-Wert von 2 - 4 eingestellt wird.

## Claims

1. Method for determining the content of catalyst fines in a sample of heavy fuel oil, in particular of a fuel for ship engines, by means of the following steps:
a) taking a sample from the heavy fuel oil,
b) in a first container, diluting the sample by means of a first reagent consisting of solvents containing mainly hydrocarbons and intensely mixing said sample therewith,
c) introducing a portion of the mixture from the first container into a second container containing diluted hydrochloric acid as a second reagent and intensely mixing said portion with the hydrochloric acid,
d) removing a portion of the contents from the second container and transferring said portion into a third container via a filter,
e) in the third container, ascertaining the content of catalyst fines in the sample by determining the strength of the resultant fluorescent radiation upon excitation by UV light,
f) measuring and evaluating the exhibited fluorescent radiation visually or with the aid of a photometer.

2. Method according to claim 1, **characterised in that** the first reagent is a standard diluent in the form of a universal cold cleaner consisting of aliphatic hydrocarbons having n-alkanes, C₁₀-C₁₃ compounds, iso-alkanes and cycloalkanes.

3. Method according to claim 1, **characterised in that** the second reagent is a 0.05 molar hydrochloric acid solution diluted with double-distilled water.

4. Method according to claim 1, **characterised in that** the process of removal from the second container takes place by means of a syringe, the contents of which are discharged via a filter wetted with diluted hydrochloric acid.

5. Method according to claim 1, **characterised in that** the mixing process according to step c) is carried out by means of a vortex mixer at ambient temperature.

6. Method according to claim 1, **characterised in that** the mixing process according to step c) is carried out by means of ultrasound at an increased temperature.

7. Method according to claim 1, **characterised in that** process of excitation by UV radiation is carried out by means of at least one UV diode at 365 nm.

8. Method according to claim 7, **characterised in that** the strength of the fluorescent radiation is determined at 470 nm.

9. Method according to claim 4, **characterised in that**, before use, the filter is pre-rinsed with a 0.05 molar hydrochloric acid solution that is pH-stabilised in the range of from 2 to 4.

10. Method according to claim 2, **characterised in that**, in step b), heavy fuel oil (HFO) is mixed with a diluent according to claim 2 in the volume ratio 2:1 for 2 to 4 minutes in the first container by means of a vortex mixer, **in that**, in step c), 5 ml of the second reagent is mixed with 0.2 ml of the mixture from the first container for 20 to 40 seconds in the second container by means of a vortex mixer, **in that**, in step d), 3 to 5 ml is removed and filtered and the filtrate is transferred into the third container, in which the fluorescent radiation is determined according to step f).

11. Method according to claim 1, **characterised in that** the filtrate removed from the second container in step c) is set to a pH of from 2 to 4.

## Revendications

1. Procédé pour déterminer la teneur en fines de catalyseur dans un échantillon de fioul lourd, en particulier d'un carburant pour moteurs de bateau, à l'aide des étapes suivantes :
a) un échantillon est prélevé dans le fioul lourd,
b) l'échantillon est dilué dans un premier récipient avec un premier réactif composé d'un diluant contenant principalement des hydrocarbures, et est mélangé énergiquement,
c) une partie du mélange du premier récipient est placée dans un deuxième récipient contenant comme deuxième réactif de l'acide chlorhydrique dilué, et est mélangée énergiquement avec l'acide chlorhydrique,
d) une partie du contenu est prélevée dans le deuxième récipient et est transférée par l'intermédiaire d'un filtre dans un troisième récipient,
e) dans le troisième récipient a lieu la constatation de la teneur en fines de catalyseur dans l'échantillon, par détermination de l'intensité du rayonnement fluorescent obtenu, en présence d'une excitation par UV,
f) le rayonnement fluorescent qui apparaît est relevé visuellement ou à l'aide d'un photomètre, et analysé.

2. Procédé selon la revendication 1, **caractérisé en ce que** le premier réactif est un diluant standard sous la forme d'un épurateur à froid universel qui se compose d'hydrocarbures aliphatiques avec des n-alcanes, des composés C₁₀-C₁₃, des iso-alcanes et des cycloalcanes.

3. Procédé selon la revendication 1, **caractérisé en ce que** le deuxième réactif est une solution d'acide chlorhydrique de concentration molaire 0,05 diluée avec de l'eau bidistillée.

4. Procédé selon la revendication 1, **caractérisé en ce que** le prélèvement dans le deuxième récipient se fait à l'aide d'une seringue à piston dont le contenu est évacué à travers un filtre humecté d'acide chlorhydrique dilué.

5. Procédé selon la revendication 1, **caractérisé en ce que** le mélange de l'étape c) est réalisé à l'aide d'un mélangeur à vortex à température ambiante.

6. Procédé selon la revendication 1, **caractérisé en ce que** le mélange de l'étape c) est réalisé à l'aide d'ultrasons à une température plus élevée.

7. Procédé selon la revendication 1, **caractérisé en ce que** l'excitation par rayonnement UV se fait à l'aide d'au moins une diode UV à 365 nm.

8. Procédé selon la revendication 7, **caractérisé en ce que** l'intensité du rayonnement fluorescent est déterminée à 470 nm.

9. Procédé selon la revendication 4, **caractérisé en ce que** le filtre, avant d'être utilisé, est pré-rincé avec une solution d'acide chlorhydrique de concentration molaire 0,05, en présence d'une stabilisation pH dans la plage de 2-4.

10. Procédé selon la revendication 2, **caractérisé en ce que** lors de l'étape b), du fioul lourd (HFO) est mélangé avec un diluant selon la revendication 2 suivant un rapport de volume de 2:1 pendant 2-4 minutes dans le premier récipient à l'aide d'un mélangeur à vortex, **en ce que** lors de l'étape (c), 5 ml du deuxième réactif dans le deuxième récipient sont mélangés avec 0,2 ml du mélange du premier récipient à l'aide d'un mélangeur à vortex pendant 20-40 secondes, **en ce que** lors de l'étape d), 3-5 ml sont prélevés et filtrés, et le filtrat est transféré dans le troisième récipient, dans lequel la détermination du rayonnement fluorescent de l'étape f) a lieu.

11. Procédé selon la revendication 1, **caractérisé en ce que** le filtrat prélevé dans le deuxième récipient lors de l'étape c) est réglé à une valeur de pH de 2-4.
